# EUROPEAN PATENT APPLICATION

(11) **EP 1 323 387 A1**
(43) Date of publication of application: **02.07.2003**
(21) Application number: 02011811.3
(22) Date of filing: 28.05.2002
(51) Int. Cl.: A61B 17/20, A61M 5/32

(54) **Medical needle assembly with needle protection**

(30) Priority: 28.12.2001 US 344126 P; 09.05.2002 US 141114
(71) Applicant: Becton, Dickinson and Company, Franklin Lakes, New Jersey 07417 (US)
(72) Inventor: Swenson, John D., Wayne, NJ 07470 (US); Caizza, Richard James, Vernon, NJ 07462 (US); Swenson, Kirk D., North Caldwell, NJ 07006 (US); Prais, Alfred Wesley, Hewitt, NJ 07421 (US); Alchas, Paul G., Wayne, NJ 07470 (US)
(74) Representative: Selting, Günther, Dipl.-Ing.

(57) **Abstract**

A shieldable unit dose needle assembly including a unit dose needle (12), a shield (140) in pivotal engagement with respect to the needle (12), and a collar (40) providing pivotal engagement between the needle (12) and the shield (140). The shield (140) is pivotally movable between a retracted position and a shielded position in which a portion of the shield encompasses the unit dose needle (12) for safety purposes. The collar (40) further includes a handle (70) for holding the needle assembly during use, which handle may include a profile (72) for accomodating a user's fingers.

## Description

### CROSS-REFERENCE TO RELATED APPLICATIONS

This application claims priority from U.S. Provisional Patent Application Serial No. 60/344,126, filed December 28, 2001, and entitled "Bifurcated Needle Assembly with Needle Shielding Provision."

### BACKGROUND OF THE INVENTION

### 1. Field of the Invention

The present invention relates to needles for use in medical procedures and, in particular, to safety shielded needles and needle assemblies for use in vaccination procedures.

### 2. Description of Related Art

Bifurcated or forked end needles are well-known for providing a simple and effective means for a doctor to administer a vaccine. During use, the bifurcated tip of the bifurcated needle is put into contact with either a dried or liquid substance, which adheres to the bifurcated needle tip. The bifurcated needle tip is then put into contact with the skin of the patient who is being administered the vaccination. The skin is either scratched or pierced with the needle tip so that the vaccination material may be absorbed into the skin of the patient. An alternative method of delivering the vaccination includes placing a drop of the vaccine onto the skin of the patient and contacting the skin of the patient with the bifurcated needle tip through the drop of vaccine. Alternatively, a standard pointed needle tip without a lumen may also be used when the drop of vaccine is applied directly to the skin of the patient.

The bifurcated needle is considered a significant medical advancement because it has allowed more people to be vaccinated with less serum. This has been especially important for those living in less developed areas because of the efficient and easy to use design, as well as the ease of replication.

Vaccination effectiveness, however, is reduced if the bifurcated needle is reused too many times. Moreover, reuse of such vaccination needles exposes patients to the risk of transmission of infectious diseases through percutaneous contact through the skin. Additionally, medical care workers using traditional vaccination needles are at an increased risk of exposure to infectious diseases due to the design of such needles, which makes them difficult to handle, as well as due to the repeated use of such needles.

In particular, bifurcated needles used to administer vaccinations are not traditionally sterilized or packaged in a single use container that would enable convenient storage and subsequent use. Additionally, such needles have traditionally been difficult to handle in that they typically do not include a hub attached to the opposite end of a needle from the tip, and do not typically include any sort of shield for protection from the needle prior to and after use.

For example, U.S. Patent No. 3,194,237 to Rubin discloses a vaccination needle having a main shank with a pair of prongs at one end that define a slot of predetermined length, width and depth therebetween to hold an amount of liquid by capillary action. The shank of the needle is of sufficient length so that the non-prong end will function as a handle. U.S. Patent No. 3,948,261 to Steiner discloses a reusable unit dose container for vaccines contained within a rigid receptacle, with a compressible closure for supporting a bifurcated needle bearing dried vaccine. The closure is adapted to support the needle in the container during a lyophilizing process while liquid vaccine is dried on the needle. The closure has grooves which permit the vaporized liquid from the vaccine to be withdrawn from the receptacle during lyophilizing, and can further seal the container.

Numerous devices have been developed in the medical field for shielding needles after use. Many of these devices are somewhat complex and costly. In addition, many of these devices are cumbersome to use in performing procedures. Furthermore, some of the devices are so specific that they preclude use of the device in certain procedures or with certain devices and/or assemblies.

For example, U.S. Patent No. 5,188,611 discloses a reusable safety needle arrangement having a collar for engaging a needle and a slotted longitudinal shield which is attached to the collar at a hinge for pivoting over the needle. Such devices incorporating a pivoting shield assembly are typically used with hypodermic syringe needles or double-ended phlebotomy needles.

In view of the foregoing, a need exists for a shieldable needle assembly for use with a unit dose vaccination needle that is easily manufactured, that is simple to use, that is easily sterilized and maintained in a sterile condition until used, that can be safely disposed of, and that does not interfere with normal practices of bifurcated needle use.

### SUMMARY OF THE INVENTION

The present invention is directed to a shieldable unit dose needle assembly for administering a unit dose of a vaccine to a patient. The shieldable assembly includes a unit dose needle having a handle end and a prong end configured to hold a unit dose of a vaccine. The shieldable assembly further includes a collar or base including a needle end, with the unit dose needle extending from the needle end of the collar, and a handle extending from the collar opposite the needle end. The handle may be integrally formed with the collar, or may be a separate piece fixedly attached to the collar. The handle preferably includes a profile for accommodating a user's fingers. A pivoting shield extends from the collar in pivotal engagement with respect to the unit dose needle, and is pivotally movable between a retracted position pivotally spaced from the prong end of the unit dose needle and a shielded position in alignment with and encompassing or enveloping the prong end of the unit dose needle.

The unit dose needle desirably is in the form of a bifurcated needle, with the prong end including at least two pointed prongs which are capable of penetrating or abrading the skin of a patient, and which are separated by a U-shaped or V-shaped channel capable of holding the unit dose of vaccine. A hub may be fixedly attached to the handle end of the unit dose needle for attaching the unit dose needle to the collar, with corresponding engaging surfaces for attachment therebetween, such as through threaded engagement. A removable packaging needle cover in the form of a semi-rigid sleeve may also be provided, which encompasses the unit dose needle when the shield is in a retracted position and which is detachably matable with the collar. Such a semi-rigid sleeve desirably provides a hermetically sealed barrier enclosing the needle therein.

The shield may be pivotally connected to the collar through a hinged connection established by a hangar bar located on the shield and a hook arm located on the collar, or through a living hinge extending between the shield and the collar.

Means for preventing pivotal movement of the shield between the shielded position and the retracted position after the shield has been pivoted to the shielded position may further be included, such as a latch arm or hook extending within the internal opening of the shield for engaging the needle, and/or corresponding locking structure between the shield and the collar, such as latches and detents. Such means may be irreversible, in that the shield cannot be moved from the shielded position without excessive force by the user.

In a further embodiment, the hub and needle form a unit dose needle assembly; and the collar, handle and shield form a shield assembly, with the unit dose needle assembly and the shield assembly being separately packaged or packaged together in a detached state, and attachable to form a shieldable needle assembly for administering a unit dose of a vaccine. For example, the unit dose needle assembly may include a hub having a needle end and an engagement end, and a unit dose needle extending from the needle end of the hub. The shield assembly may include a collar having a needle engagement end and a handle end, a shield pivotably connected to the collar, and a handle attached to the handle end of the collar. The needle engagement end of the collar and the engagement end of the hub include corresponding structure such as corresponding threaded surfaces for attachment of the unit dose needle assembly with the shield assembly, thereby forming the shieldable needle assembly. The shield is pivotal with respect to the unit dose needle between a retracted position pivotally spaced from the unit dose needle and a shielded position with the unit dose needle encompassed within the shield. The shield may be pivotally connected to the collar through a hangar bar located on the shield and a hook arm located on the collar, or may be pivotally connected to the collar through a living hinge extending between the shield and the collar.

In a further embodiment, the present invention is directed to a shieldable, single use unit dose needle assembly for administering a unit dose of a vaccine which includes a unit dose needle having a handle end and a prong end, a fitting capable of receiving the unit dose needle and including a hinge attached to an outer surface of the fitting, a handle extending from the fitting, and an elongated housing attached to the hinge. The elongated housing is pivotal and capable of enclosing the unit dose needle, and includes a longitudinal slot for receiving the unit dose needle and at least one elongated door member hingedly attached to the housing extending across the longitudinal slot over substantially the entire length of the longitudinal slot.

### DESCRIPTION OF THE DRAWINGS

FIG. 1 is a perspective view of the shieldable unit dose needle assembly of the present invention including related packaging features;

FIG. 2 is a perspective view of the unassembled pieces of FIG. 1;

FIG. 3 is a bottom view of the shield as shown in FIG. 2;

FIG. 4 is a cross-sectional view of the collar as shown in FIG. 2 taken along lines 4-4 thereof;

FIG. 5 is a cross-sectional view of the needle hub as shown in FIG. 2 taken along lines 5-5 thereof;

FIG. 6 is a cross-sectional view of the shield as shown in FIG. 2 taken along lines 6-6 thereof;

FIG. 7 is a cross-sectional side view of the shieldable assembly of FIG. 1;

FIG. 8 is a perspective view of the shieldable assembly of FIG. 1 with the needle packaging cover sleeve removed and the shield in a retracted position;

FIG. 9 is a cross-sectional side view of the shieldable assembly of FIG. 1 shown with the needle packaging cover sleeve removed and the shield in a retracted position;

FIG. 10 is a cross-sectional side view of the shieldable assembly of FIG. 1 with the needle packaging cover sleeve removed and the shield in a fully shielded position;

FIG. 11 is a cross-sectional view of a unit dose needle assembly for use with a shieldable assembly in accordance with an alternate embodiment of the present invention;

FIG. 12 is a cross-sectional view of a collar for engagement with the unit dose needle assembly of FIG. 11;

FIG. 13 is a cross-sectional side view of a shieldable assembly including the unit dose needle assembly of FIG. 11;

FIG. 14 is a cross-sectional view of an alternate unit dose needle assembly for use in a shieldable assembly in accordance with a further embodiment of the present invention;

FIG. 15 is a cross-sectional view of a collar for engagement with the unit dose needle assembly of FIG. 14;

FIG. 16 is a cross-sectional side view of a shieldable assembly including the unit dose needle assembly of FIG. 14;

FIG. 17 is a cross-sectional view of a further alternate unit dose needle assembly for use in a shieldable assembly in accordance with a further embodiment of the present invention;

FIG. 18 is a cross-sectional view of a collar for engagement with the unit dose needle assembly of FIG. 17;

FIG. 19 is a cross-sectional side view of a shieldable assembly including the unit dose needle assembly of FIG. 17;

FIG. 20 is a side-sectional view of a shieldable assembly in a further embodiment of the present invention, showing the unit dose needle separated from the shield assembly;

FIG. 21 is a cross-sectional view taken along line 21-21 of FIG. 20; and

FIG. 22 is a side view of a shieldable assembly in yet a further embodiment of the present invention, showing the rigid packaging cover separated from the needle.

### DETAILED DESCRIPTION

While this invention is satisfied by embodiments in many different forms, there is shown in the drawings and will herein be described in detail, the preferred embodiments of the invention, with the understanding that the present disclosure is to be considered as exemplary of the principles of the invention and is not intended to limit the invention to the embodiments illustrated. Various other modifications will be apparent to and readily made by those skilled in the art without departing from the scope and spirit of the invention. The scope of the invention will be measured by the appended claims and their equivalents.

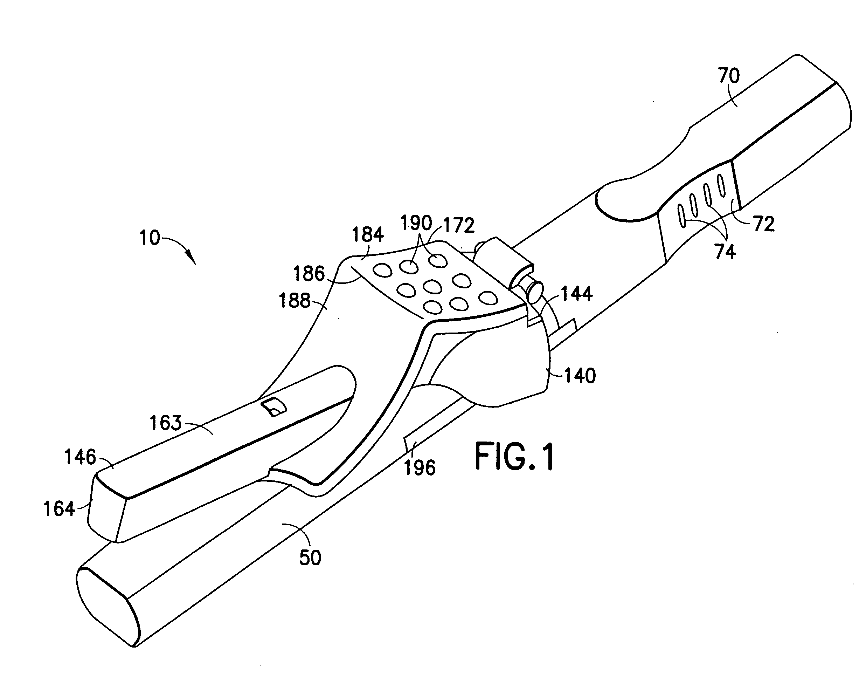

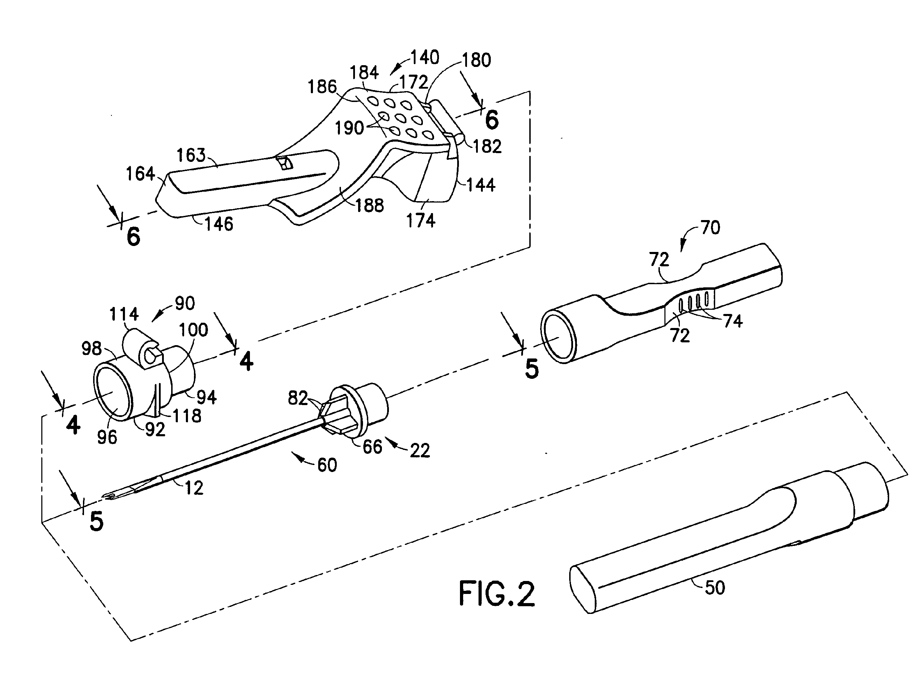

Referring to the drawings in which like reference characters refer to like parts throughout the several views thereof, FIGS. 1 and 2 illustrate a shieldable unit dose needle assembly **10** in accordance with the present invention, and the related packaging features. The shieldable needle assembly **10** includes a unit dose needle such as a bifurcated needle **12** and a hub **22,** which together form a single use, unit dose needle assembly **60**. The shieldable needle assembly **10** further includes a safety shield assembly including a collar **90,** a housing in the form of a pivotable shield **140,** and a handle **70**.

The needle assembly **10** of the present invention is intended for use for the administration of vaccines applied to or through the skin of the patient, and is intended as a single use vaccination needle assembly including features to maintain sterility of the needle during packaging, and to provide safety shielding for the medical practitioner after use, as will be described in more detail herein.

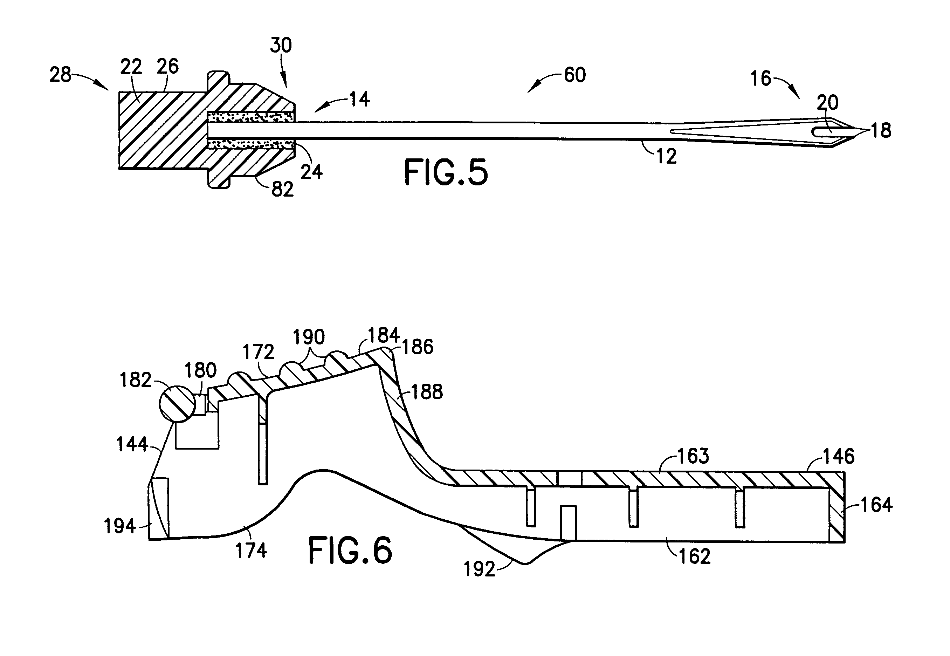

The needle assembly **10** includes a unit dose needle assembly **60,** as shown in FIGS. 2 and 5. The unit dose needle assembly **60** generally includes a unit dose needle for administering a unit dose of a vaccine, such as a bifurcated needle **12,** which is supported by a hub **22**. While needle assembly **10** is described herein in terms of a preferred embodiment including a bifurcated needle **12** as the unit dose needle, needle assembly **10** may include any unit dose needle capable of administering a unit dose of a vaccine, such as in a lyophilized dry form or liquid form, as is well-known in the art.

The bifurcated needle **12** includes a handle end at proximal end **14,** and an opposed prong end at distal end **16.** Bifurcated needle **12** is provided with two sharp prongs **18** positioned at a distal end **16** of the needle. The prongs **18** are separated by a U-shaped channel **20** configured to hold a unit dose of vaccine. The prongs **18** are intended to penetrate or abrade the skin of the patient to administer the vaccine disposed in the U-shaped channel **20.** Bifurcated needle **12** may be constructed of any material known in the art, such as metal or plastic, and is desirably constructed of a medical grade surgical steel.

Needle assembly **10** may further include a hub **22** fixedly attached to the proximal end **14** of bifurcated needle **12,** such as through an adhesive joint **24.** Adhesive joint **24** may be provided through any adhesive capable of fixedly attaching or adhering bifurcated needle **12** to hub **22,** such as an oven or U.V. cured epoxy or equivalent adhesive. Hub **22** includes a hub housing **26** including a proximal end **28** and a distal end **30.** Desirably, distal end **30** of hub **22** includes an internal bore having an internal diameter of approximately the same size as or a slightly larger size than the outer diameter of the proximal end **14** of bifurcated needle **12,** for accommodating and fixedly adhering bifurcated needle **12** within such an internal bore of hub **22.**

As noted above, unit dose needle assembly **60** including bifurcated needle **12** and hub **22** are interengaged with a safety shield assembly, thus providing a shieldable feature for bifurcated needle **12** after use. As shown in FIGS. 1 and 2, this shieldable feature is achieved through a shield assembly including collar **90,** shield **140,** and handle **70.** Collar **90** acts as a fitting for mating shield **140** and handle **70** with bifurcated needle **12** through hub **22.**

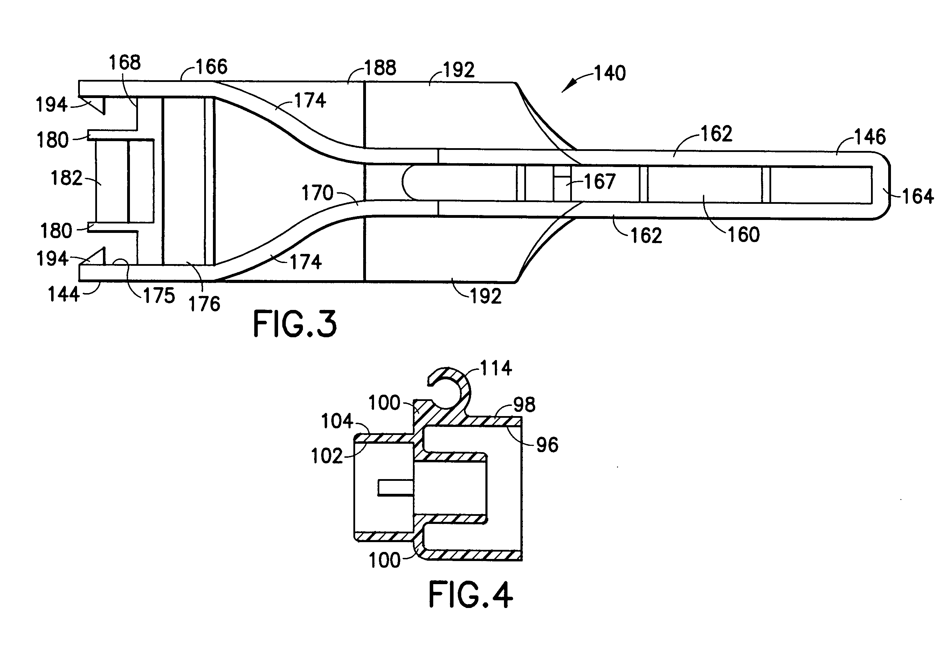

As shown in FIGS. 2 and 4, collar **90** may include two sections, a forward annular skirt **92** at a distal end thereof, and a rearward annular skirt **94** at a proximal end thereof. The forward annular skirt **92** is cylindrical, including an inner sidewall **96** and an outer sidewall **98**, and mates with the rearward annular skirt **94** at a shoulder **100.** Rearward annular skirt **94** is cylindrical, including an inner sidewall **102** and an outer sidewall **104,** and extends from shoulder **100** opposite of forward annular skirt **92.** The inner diameter of forward annular skirt **92** is larger than the inner diameter of rearward annular skirt **94.** Alternatively, the inner diameters for collar **90** can be formed as a constant inner diameter.

Extending on outer sidewall **98** of forward skirt section **92** is a hook member **114,** and located opposite or downwardly of hook member **114** on outer sidewall **98** are latches in the form of locking dents or protrusions **118.**

Collar **90** further includes handle **70** extending from the proximal end thereof adjacent rearward annular skirt **94**. Handle **70** may be integrally formed with collar **90,** or may be a distinct and separate piece as shown in FIG. 2, which is force fitted and affixed onto outer sidewall **104** of rearward annular skirt **94** of collar **90,** such as with an adhesive, solvent welding, ultrasonic welding, snap fit, or other equivalent method. Handle **70** may be of a solid construction, or may be hollow with an internal cavity. In such an embodiment, bifurcated needle **12** may extend entirely through hub **22** and into the hollow internal cavity of handle **70**, which may facilitate manufacturing and assembling of the needle assembly **10.**

Handle **70** provides a medical practitioner with a surface area for grasping and using needle assembly **10** during administration of a vaccine, as will be discussed in more detail herein. Accordingly, handle **70** includes a surface area capable of accommodating a practitioner's fingers for use, and is therefore desirably somewhat elongated in structure. The length of the handle **70** is optimized to provide beneficial ergonomic conditions for administering the vaccination or performing other medical procedures utilizing the bifurcated needle **12.** Additionally, handle **70** desirably includes a specific profile for accommodating a user's fingers, such as arcuate surfaces **72** extending along opposing sides of handle **70.** In addition or instead of such arcuate surfaces **72,** handle **70** may include structure for effectively grasping needle assembly **10,** such as ribs **74** extending along opposing sides of handle **70.** Desirably, handle **70** includes such ribs **74** along the arcuate surfaces **72,** as shown in FIG. 1.

As shown in FIGS. 2, 3 and 6, shield **140** comprises a rearward end **144** and a forward end **146**. Forward end **146** of shield **140** includes a slot or longitudinal opening **160** formed by sidewalls **162** that extend downwardly from top section **163** and run substantially opposite of one another in parallel along the length of slot **160** toward forward end sidewall **164**. Means for trapping and retaining a needle in slot **160** may be provided in the form of an arm **167** that is located at one of sidewalls **162** to secure the used needle.

Arm **167** is deflectable by needle **12** when the needle **12** enters slot **160.** Once needle **12** passes the end of arm **167**, arm **167** moves back to its original position, whereby needle **12** is permanently trapped in slot **160** by arm **167.**

At rearward end **144** of shield **140** is a collar engaging area **166** that is a continuation of slot **160.** Collar engaging area **166** includes a rearward end **168**, a forward end **170,** a top finger guide area **172,** parallel sidewalls **174** that extend downwardly and inwardly from top finger guide area **172** and into sidewalls **162,** an underside area **176** for surrounding collar **90,** and extending arms **180** to hold hanger bar **182**. Parallel sidewalls **174** include an inner surface **175** where detents such as barb dents **194** are located.

Top finger guide area **172** comprises a first ramp **184** that extends slightly on an upward slope from the rearward end of collar **90** engaging area to a shoulder **186.** From shoulder **186** extends a second ramp **188** which slopes downwardly toward top section **163.** Most preferably, first ramp **184** comprises touch bumps **190.** Touch bumps **190** provide a tactile and visual guide to alert the user that the user's finger has contacted shield **90** and that the shield is in a defined or controlled position. Touch bumps **190** may be any configuration so long as they extend and are distinct from top finger guide area **172.** Touch bumps **190** may also be of a distinguishing color as compared to top finger guide area **172** or shield **140.**

Second ramp **188** has interior surface **192** for urging needle **12** toward the center of slot **160** as shield **140** is being rotated into the closed position. The exterior surfaces are slightly inclined and extend radially from second ramp **188.** The interior surfaces are especially helpful if the longitudinal axis of needle **12** is misaligned with respect to the longitudinal axis of hub **22.**

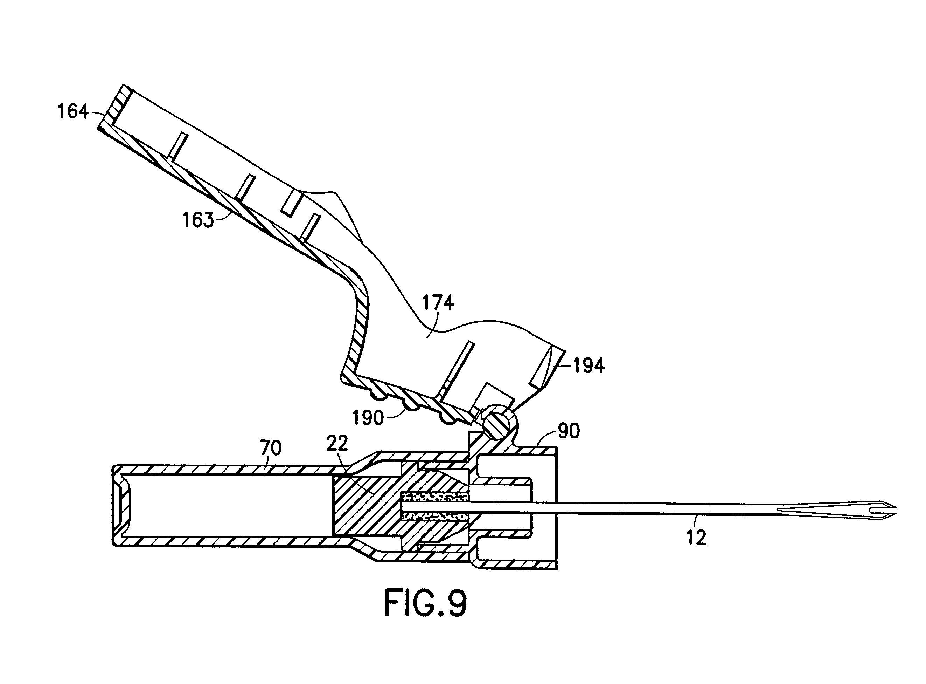

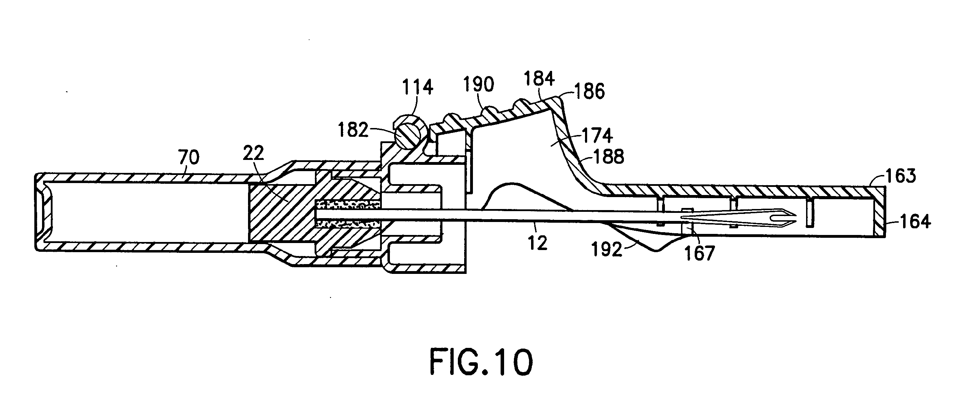

Extending arms **180** are located at rearward end **168** and at the beginning of top finger area **172** and hold hanger bar **182.** Hanger bar bis provided for pivotal engagement with hook member **114** of collar **90**. Accordingly, the cooperating surfaces of hanger bar **182** and hook member **114** are designed so as to permit rotational or pivotal movement of shield **140** with respect to collar **90.** Such engagement between hanger bar **182** and hook member **114** provides for pivotal movement of shield **140** between a retracted position as shown in FIG. 9, with shield **140** pivotally spaced from bifurcated needle **12,** and a shielded position as shown in FIG. 10, with shield **140** encompassing bifurcated needle **12.**

Located downwardly from extending arm **180** and hanger bar **182** and on inner surface **175** of parallel sidewalls **174** are barb dents **194**. Barb dents **194** cooperate with locking dents **118** on collar **90** to secure shield **140** in its final locked or shielded position.

The safety shield assembly and the unit dose needle assembly are assembled together, whereby bifurcated needle **12** is connected to hub **22** and sealed with adhesive at adhesive joint **24.** Hub **22** is then joined with collar **90** in either a fixed or non-fixed manner. Hub **22** can be fixedly joined with collar **90** bysuch techniques such as ultra-sonic welding techniques or any other bonding techniques, or mechanical fit, whereby rearward annular skirt **94** of collar **90** may be mated with hub **22.** Hub **22** may be contained or force fitted within inner sidewall **102** of rearward annular skirt **94** of collar **90.** Collar **90** is aligned with distal end **16** of bifurcated needle **12.** Then a packaging needle cover **50** which may be in the form of a semi-rigid sleeve is force fitted into inner sidewall **96** of forward annular skirt **92** of collar **90** to cover bifurcated needle **12.** Alternatively, needle cover **50** and collar **90** may include interengaging structure for mating therebetween, such as corresponding threaded surfaces for threaded engagement therebetween or slight interference or friction fits therebetween. Thereafter, shield **140** is connected to collar **90** whereby hanger bar **182** is force fitted into hook member **114** with slot **160** facing needle cover **50**. Most preferably, shield **140** is connected to collar **90** by a force fit or interface fit between hanger bar **182** and hook member **114.** Therefore, shield **140** is always oriented in a stable position and will not move unless movement of the shield **140** is positively initiated by the user. Shield **140** can then be moved toward needle cover **50** for a low profile packaged product. In addition, a label **196** may be applied to the finally assembled parts. The label **196** may be used to provide tamper evidence, thereby prevent tampering of the parts, so that they are not reused.

During assembly and packaging, the needle assembly may be subjected to a sterilization process, such as e-beam, cobalt, or ethylene oxide sterilization processes, as are well known in the art. Needle cover **50** provides a hermetically sealed barrier enclosing bifurcated needle **12** in a sterile environment therein.

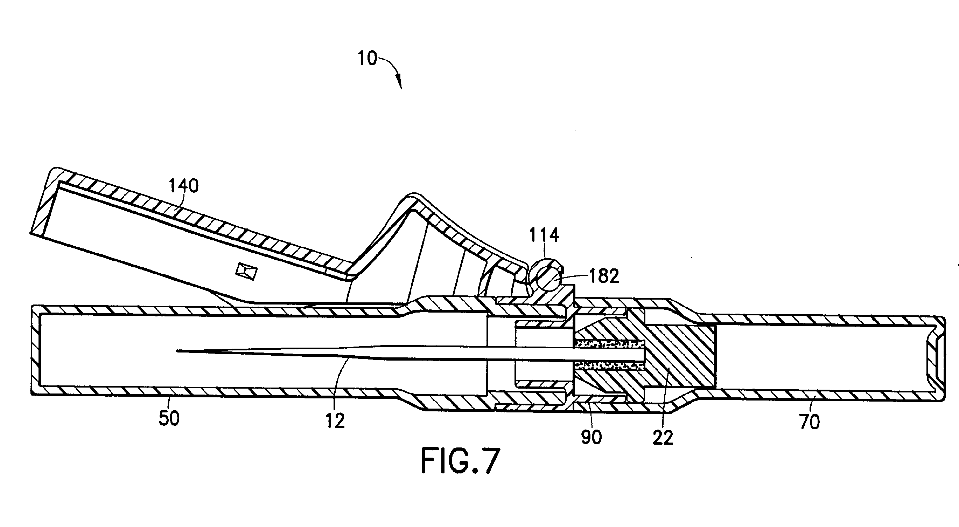

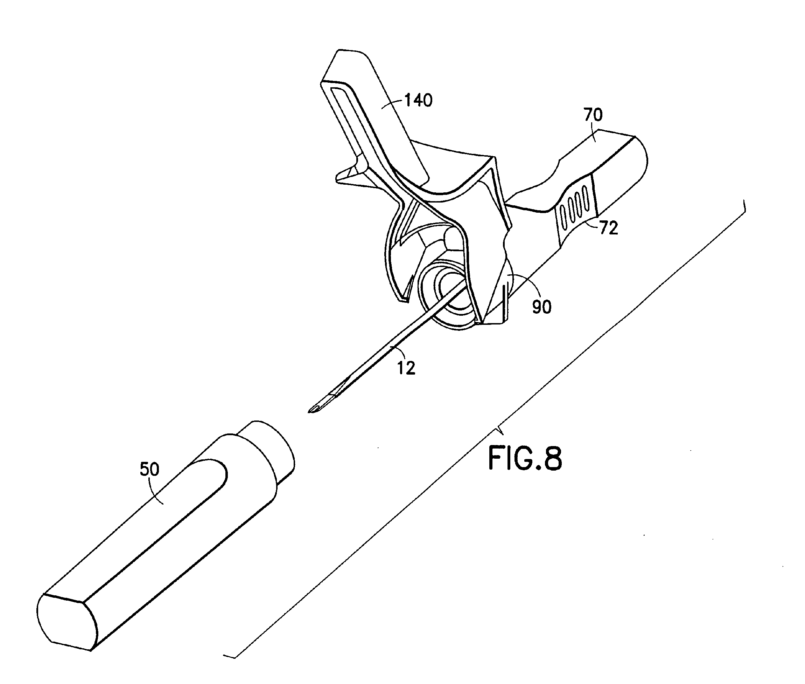

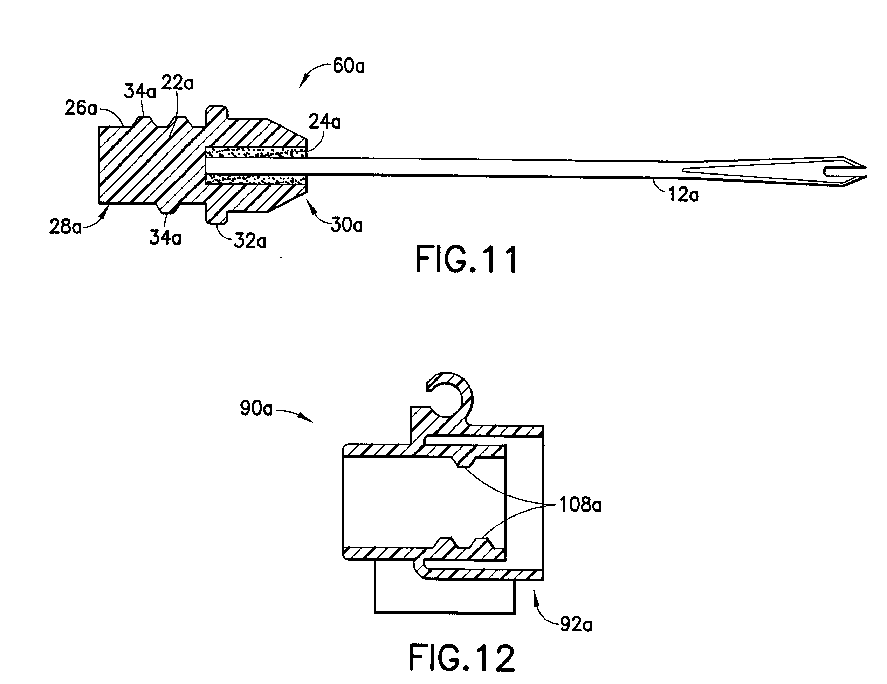

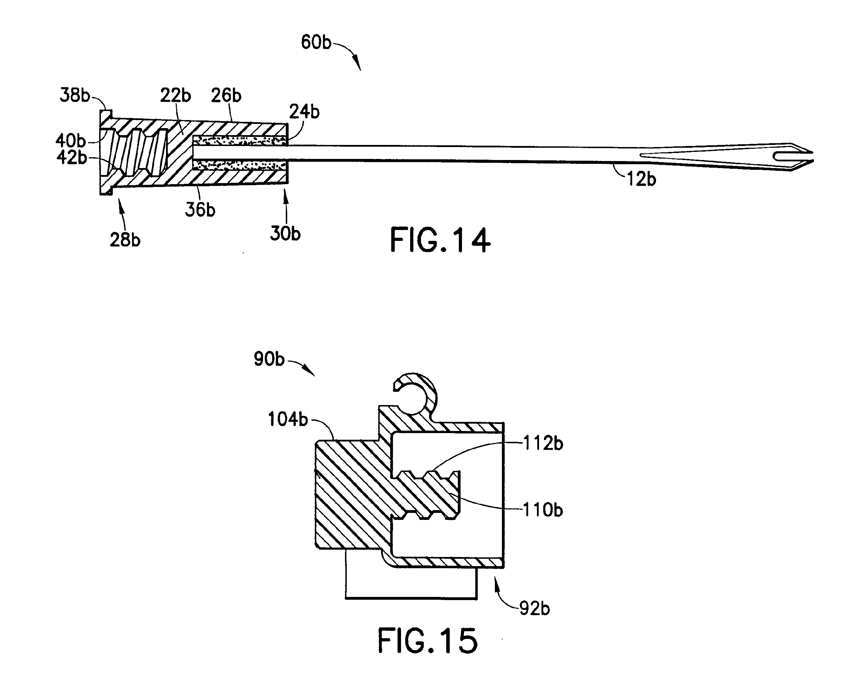

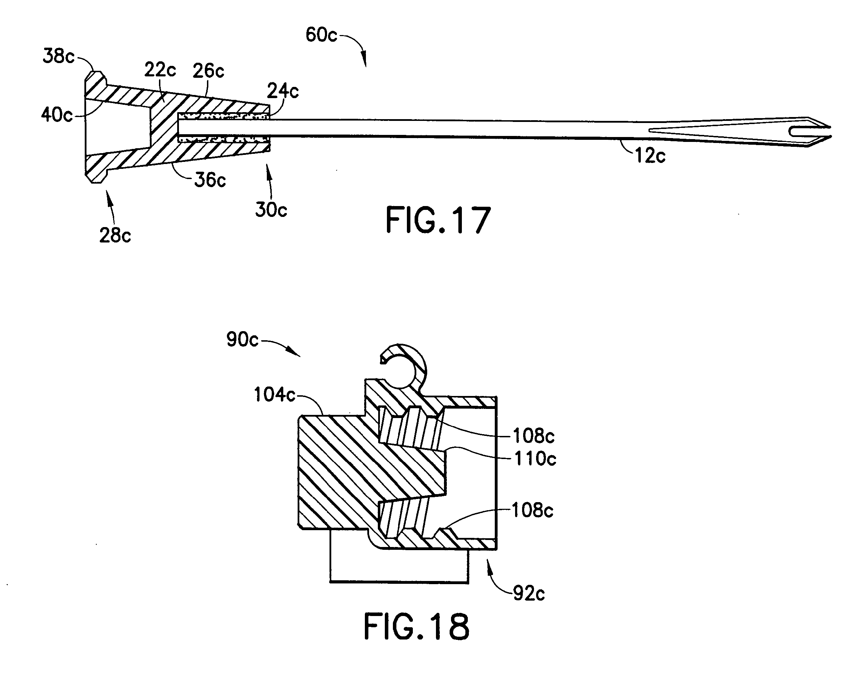

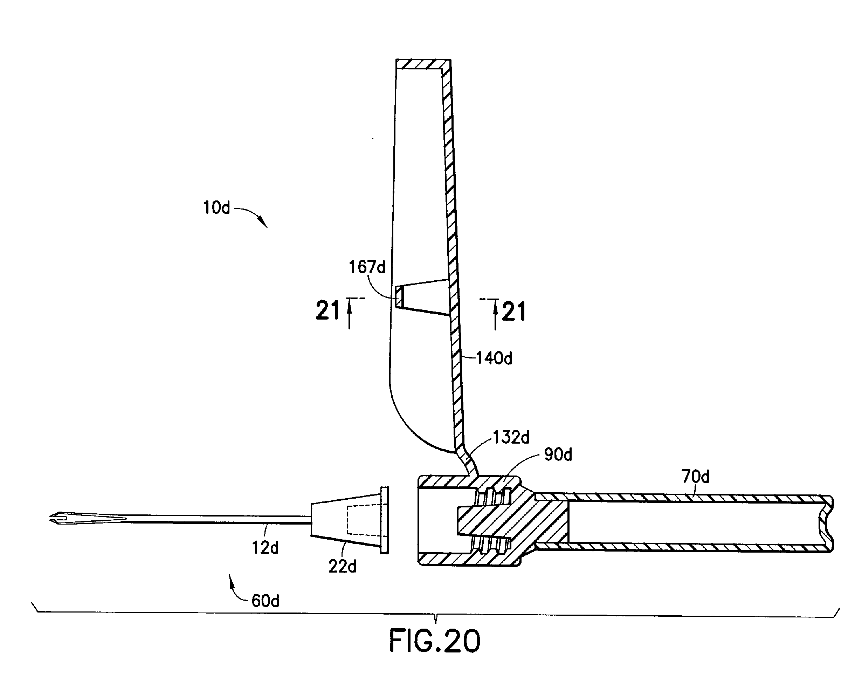

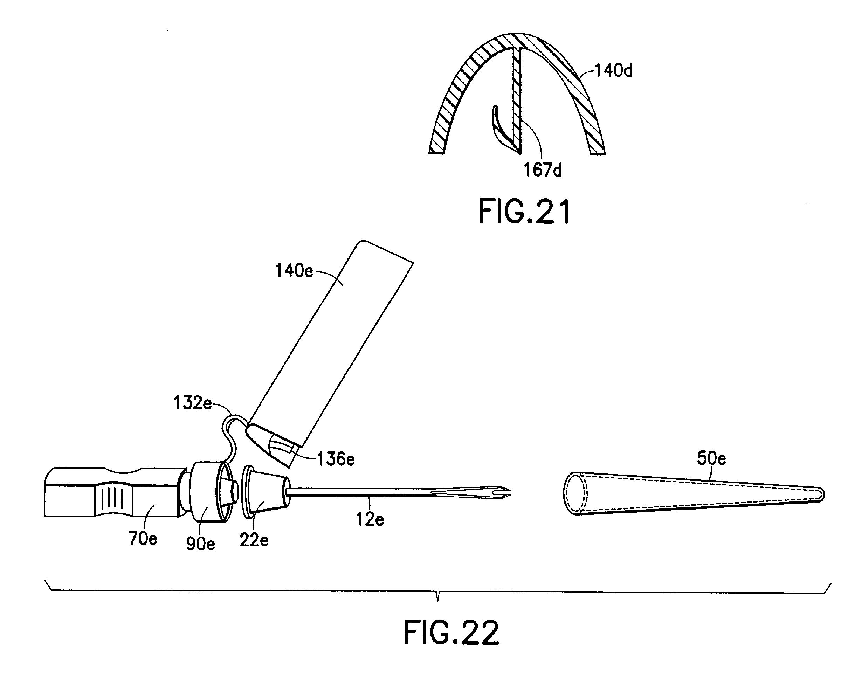

FIGS. 11-22 depict further embodiments of the present invention that include many components which are substantially identical to the components of FIGS. 1-10. Accordingly, similar components performing similar functions will be numbered identically to those components of FIGS. 1-10, except that a suffix "a" will be used to identify those similar components in FIGS. 11-13, a suffix "b" will be used to identify those similar components in FIGS. 14-16, a suffix "c" will be used to identify those similar components in FIGS. 17-19, a suffix "d" will be used to identify those similar components in FIGS. 20-21, and a suffix "e" will be used to identify those similar components in FIG. 22.

FIG. 11 depicts an alternate embodiment of a unit dose needle assembly **60a** for use with a shieldable needle assembly in accordance with the present invention. In the embodiment of FIG. 11, hub **22a** includes a hub housing **26a** including a proximal end **28a** and a distal end **30a** separated by flange **32a.** Bifurcated needle **12a** extends from distal end **30a** of hub **22a,** and is affixed thereto through adhesive joint **24a.** Proximal end **28a** of hub **22a** further includes external threads **34a** for providing interengagement with collar **90a.**

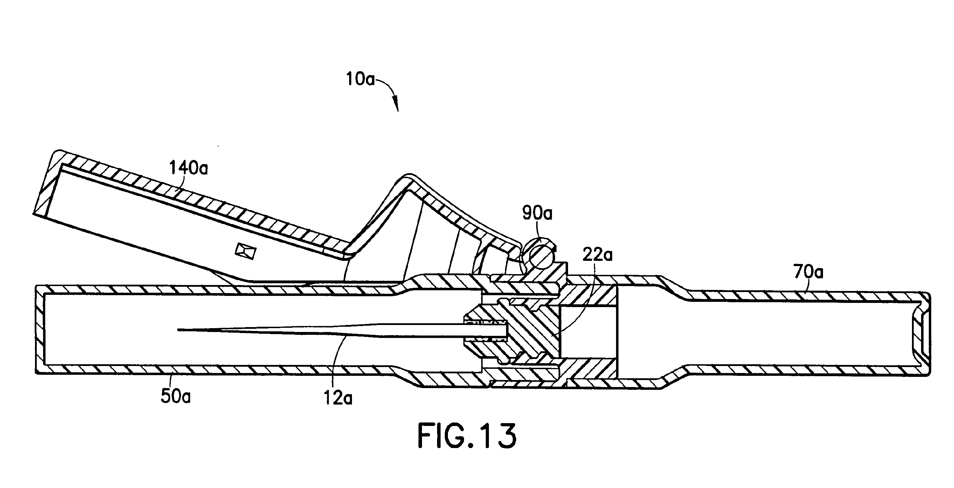

More particularly, as shown in FIGS. 12 and 13, collar **90a** desirably includes internal threads **108a** extending within forward annular skirt **92a.** Internal threads **108a** of collar **90a** and external threads **34a** of hub **22a** provide interengaging threaded structure between collar **90a** and unit dose needle assembly **60a,** thereby providing a means for attaching unit dose needle assembly **60a** to collar **90a** to provide a shielding feature. As such, unit dose needle assembly **60a** can be provided as a separate structure which can be attached to a separate shielding structure in the form of a shield assembly including collar **90a,** shield **140a** and handle **70a** by threading external threads **34a** with internal threads **108a** of collar **90a,** thereby providing a shieldable needle assembly **10a** as shown for use in FIG. 13.

FIG. 14 depicts a further embodiment of a unit dose needle assembly **60b** for use with a shieldable needle assembly **10b** in accordance with the present invention. In the embodiment of FIG. 14, hub **22b** includes a hub housing **26b** including a proximal end **28b** and a distal end **30b,** with bifurcated needle **12b** extending from and affixed to distal end **30b** through adhesive joint **24b.** The external surface of hub housing **26b** may define an outer tapered surface **36b** extending therealong. Proximal end **28b** of hub **22b** further includes a full or partial hub rim **38b** extending fully or partially circumferentially about the proximal end thereof, with an internal luer taper **40b** extending internally within a portion of hub housing **26b.** Internal luer taper **40b** may further include internal threads **42b** for providing threaded interengagement with collar **90b**.

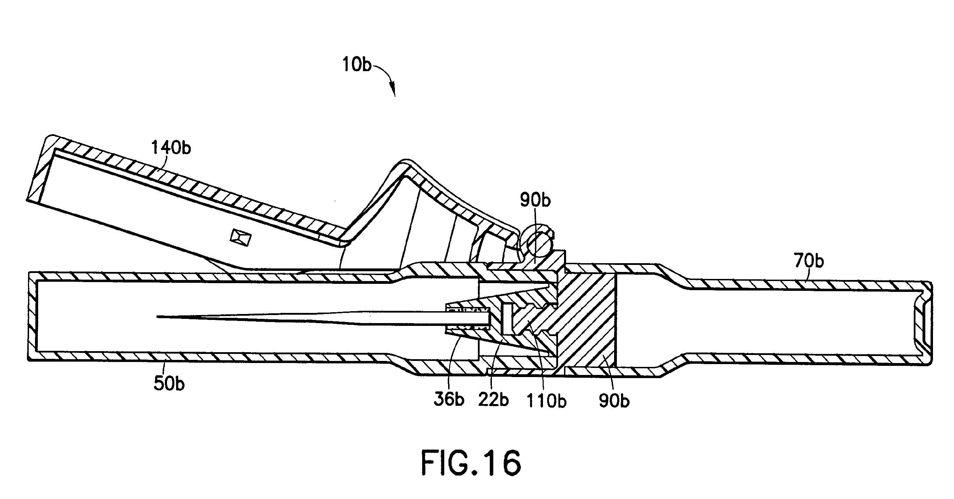

As shown in FIGS. 15 and 16, collar **90b** includes a nub **110b** having external threads **112b** extending thereabout for threaded engagement with internal threads **42b** of hub **22b**, providing interengaging threaded structure therebetween in a similar manner as with the assembly described in FIGS. 11-13. Handle **70b** is affixed to outer sidewall **104b,** thereby providing a separate shielding structure in the form of a shield assembly including collar **90b,** shield **140b** and handle **70b** for attachment with unit dose needle assembly **60b.** In such an arrangement, needle cover **50b** desirably mates with outer tapered surface **36b** of hub **22b,** within forward annular skirt **92b** of collar **90b.**

FIGS. 17-19 depict yet a further embodiment of a unit dose needle assembly **60c** for use with a shieldable needle assembly **10c** in accordance with the present invention. In this embodiment, hub **22c** includes a hub housing **26c** including a proximal end **28c** and a distal end **30c**, with bifurcated needle **12c** extending from and affixed to distal end **30c** through adhesive joint **24c.** The external surface of hub housing **26c** defines an outer tapered surface **36c** extending therealong. Proximal end **28c** of hub **22c** further includes luer lugs or a hub rim **38c** extending fully or partially circumferentially about the proximal end thereof, with an internal luer taper **40c** extending internally within a portion of hub housing **26c**.

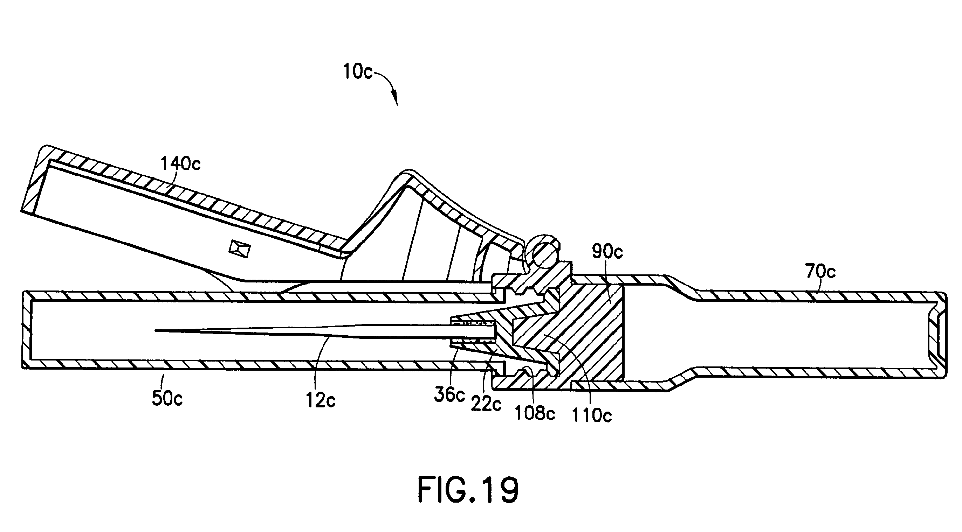

As shown in FIGS. 18 and 19, collar **90c** includes a tapered nub **110c,** having a profile for mating with the internal surface of internal luer taper **40c** of hub **22c.** In addition, collar **90c** preferably includes internal threads **108c** extending within forward annular skirt **92c.** Internal threads **108c** of collar **90c** mate with hub rim **38c** of hub **22c,** thereby providing interengaging threaded structure between collar **90c** and unit dose needle assembly **60c,** for attaching unit dose needle assembly **60c** to collar **90c** to provide a shielding feature. Handle **70c** is affixed to outer sidewall **104c.** In such a structure, needle cover **50c** desirably mates with outer tapered surface **36c** of hub **22c,** within forward annular skirt **92c** of collar **90c.** Alternatively, needle cover **50c** may include an annular rim extending circumferentially about the end thereof, for threaded engagement with internal threads **108c** of collar **90c** after hub **22c** has been mated therewith.

FIGS. 20 and 21 depict a unit dose needle assembly **60d** in combination with a shield assembly including a living hinge **132d** extending between collar **90d** and shield **140d**. Living hinge **132d** permits shield **140d** to pivot between the retracted position and the shielded position, as discussed with respect to the above embodiments. Living hinge **132d**, collar **90d,** shield **140d,** and handle **70d** can be integrally molded and formed as a single shielding structure to form a shield assembly. Unit dose needle assembly **60d** can then be attached to such a shield assembly, thereby forming needle assembly **10d.** Shield **140d** may further include arm **167d,** which acts as a locking mechanism with bifurcated needle **12d** in a similar manner as described above. Additionally, in an embodiment where living hinge **132d,** collar **90d,** shield **140d,** and handle **70d** are integrally molded, bifurcated needle **12d** can be assembled through bonding means to a bore (not shown) in the collar to provide an easier to manufacture assembly. Additionally, it is contemplated that bifurcated needle **12d** can be integrally molded as an extension from the collar when made from similar moldable materials.

In FIG. 22, a unit dose needle assembly **60e** is shown in combination with a shield assembly including a living hinge **132e,** with a locking mechanism in the form of an elongated door **136e** on shield **140e.** Elongated door **136e** acts as a locking mechanism with bifurcated needle **12e** in a similar manner as described above with respect to arm **167** acting as a means for trapping bifurcated needle **12.** Desirably, elongated door **136e** extends over substantially the entire length of the longitudinal slot of shield **140e.** Elongated door **136e** is biased to close the longitudinal slot after shield **140e** has been pivoted about living hinge **132e** and bifurcated needle **12e** is encompassed in shield **140e.** Desirably, elongated door **136e** is in the form of a trap door extending from a first sidewall of shield **140e** to a second sidewall of shield **140e,** with the trap door abutting a stop on the second sidewall. A pair of elongated doors may be alternatively provided, each extending from a sidewall of the housing of shield **140e,** and with the doors overlapping to close the housing. Desirably, the elongate door member is attached to the shield **140e** by a resilient living hinge.

In use, shieldable needle assembly **10** is provided as shown in FIG. 1 for use in administering a vaccine to a patient. Alternatively, unit dose needle assembly **60a, 60b, 60c, 60d** or **60e** may be provided for attachment to a shield assembly including collar **90a, 90b 90c, 90d** or **90e,** shield **140a, 140b, 140c, 140d** or **140e** and handle **70a, 70b, 70c, 70d,** or **70e,** respectively, by threadably engaging the corresponding threaded surfaces of the respective hub and collar.

The user then grasps needle assembly **10** with handle **70** between finger and thumb at arcuate surfaces **72.** Shield **140** is then rotated back by the user toward the handle **70.** Then, as shown in FIG. 11, needle cover **50** is removed from the bifurcated needle **12.** Needle assembly **10** can then be used for administration of a vaccine through the skin of a patient, using handle **70** as a handle for holding the assembly during use. For example, a unit dose of a vaccine contained within U-shaped channel **20** may be administered percutaneously to the patient by way of bifurcated needle **12.** The unit dose of the vaccine may be contained within U-shaped channel **20** during packaging and prior to removal of needle cover **50,** or the unit dose of the vaccine may be placed within U-shaped channel **20** after removal of needle cover **50** immediately prior to administration such as by accessing a vial containing multiple doses in liquid form where submersion of the U-shaped channel **20** into the vaccine retains the vaccine during removal of the bifurcated needle **12** from the vial. To administer the vaccine, the pointed prongs of bifurcated needle **12** penetrate the stratum corneum layer of the skin and deliver the vaccine contained within U-shaped channel **20** to the deep epidermis.

After administration of the vaccine is complete, the user easily pivotally rotates shield **140** from the open or retracted position toward bifurcated needle **12** to an intermediate position and then the user pushes on shield **140** at the top finger guide area **172** to move shield **140** into a final, non-retractable shielded position whereby needle **12** is trapped in longitudinal opening **160.**

During pivotal rotation of shield **140** to the shielded position, barb dents **194** on inner surface **175** of parallel sidewalls **174** of shield **140** deflect over and are held by locking dents **118** of collar **90.** The interengagement between barb dents **194** and locking dents **118** provide a locking structure for locking engagement between shield **140** and collar **90**, thereby locking shield **140** in the shielded position and preventing pivotal rotation of shield **140** to the open or retracted position. Such locking further provides a tactile feel to the user that shield **140** has been rotated to the shielded position. Alternatively, it is contemplated that shield may include a latch or locking dent and the collar may include a detent or a barb dent for providing means for locking the shield in the shielded position.

Moreover, in embodiments including a needle locking mechanism such as a hook or arm **167,** the needle snaps past arm **167** and is trapped when bifurcated needle **12** is contained within shield **140** as shield **140** is pivoted into the closed or shielded position. Alternatively, a gel material may be located in the shield near arm **167** so that when bifurcated needle **12** snaps past arm **167**, it will come to rest within the gel material.

The means for locking, whether provided through the barb dent and latch protrusion of the shield and collar, through the needle locking mechanism of the hook attaching to the needle, or through both such features, is preferably irreversible, in that once the shield is pivoting to the shielding position and locked in place, it cannot be pivoted away to expose the needle without excessive force or displacement by the user.

The shieldable needle assembly of the present invention provides for a single use unit dose application of a vaccine. The needle assembly can be packaged as a sterile assembly for single use. The needle assembly can be packaged in an appropriate box and shelf carton as required for storage and shipment. Alternatively, the unit dose needle assembly and the shield assembly can be packaged separately in sterile packaging, and assembled just prior to use by the medical practitioner.

The shield, collar, handle and hub of the safety shield assembly of the present invention are comprised of moldable parts which can be mass produced from a variety of materials such as one or more moldable plastics including, for example, polyethylenes, polypropylenes, polyamides, polyesters, fluorinated polyethylenes, polyvinyl chloride, polystyrene, and the like. Materials will be selected which will provide the proper covering and support for the structure of the invention in its use, but which will also provide a degree of resiliency for the purpose of providing the cooperative movement relative to the shield and the collar of the assembly.

Desirably, the shield, collar, handle and hub are constructed of rigid polymeric materials, thereby providing a "hardpack" configuration to the needle assembly. This "hardpack" configuration provides the benefits of a sterile barrier without requiring additional packaging. The inventive assembly also provides the benefit of an individual sterile package, which has in the past typically required paper packaging in a pouch or blister-type package. Further, bifurcated needles have traditionally been multiple use products which are re-sterilized in between uses. The hardpack configuration provides the benefit of a single use application and a sterile package in combination.

## Claims

1. A shieldable unit dose needle assembly for administering a unit dose of a vaccine comprising:
a unit dose needle having a handle end and a prong end configured to hold a unit dose of a vaccine;
a shield in pivotal engagement with respect to said unit dose needle and pivotally movable between a retracted position pivotally spaced from said prong end of said unit dose needle and a shielded position encompassing said prong end of said unit dose needle;
a collar including a needle end, said unit dose needle extending from said needle end of said collar, said collar providing for pivotal movement of said shield between said retracted position and said shielded position; and
a handle extending from said collar opposite said needle end.

2. The needle assembly of claim 1, wherein the unit dose needle comprises a bifurcated needle, wherein the prong end includes two pointed prongs which are capable of penetrating or abrading the skin of a patient, and wherein the prongs are separated by a U-shaped channel capable of holding the unit dose of a vaccine.

3. The needle assembly of claim 2, wherein the unit dose of a vaccine is a liquid.

4. The needle assembly of claim 1, further comprising a hub fixedly attached to the handle end of the unit dose needle for attaching the unit dose needle to the collar.

5. The needle assembly of claim 4, wherein the collar and the hub include corresponding engaging surfaces for attachment therebetween.

6. The needle assembly of claim 5, wherein the collar and the hub include corresponding engaging surfaces for threaded engagement therebetween.

7. The needle assembly of claim 1, wherein the handle comprises a separate piece fixedly attached to the collar.

8. The needle assembly of claim 1, wherein the handle includes a profile for accommodating a user's fingers.

9. The needle assembly of claim 1, wherein said shield further comprises tactile and visual means for providing the user with a guide for pivoting said shield.

10. The needle assembly of claim 9, wherein said means for guiding the user's fingers to move said shield into various positions is a top finger guide area comprising a first ramp that extends slightly on an upwardly slope from a rearward end of said shield to a shoulder.

11. The needle assembly of claim 10, wherein said first ramp comprises touch bumps.

12. The needle assembly of claim 1, further comprising a removable packaging needle cover which encompasses said unit dose needle when said shield is in a retracted position.

13. The needle assembly of claim 1, wherein the collar, shield and handle each comprise one or more moldable plastics.

14. The needle assembly of claim 13, wherein the plastics are one or more selected from the group consisting of polyethylenes, polypropylenes, polyamides, polyesters and fluorinated polyethylenes.

15. The needle assembly of claim 1, further comprising means for preventing pivotal movement of said shield between the shielded position and the retracted position after the shield has been pivoted to the shielded position.

16. The needle assembly of claim 1, wherein the shield is pivotally connected to the collar through a hangar bar located on said shield and a hook arm located on said collar whereby said hangar bar engages with said hook arm so that said shield may be pivoted with respect to said collar between said retracted position and said shielded position.

17. The needle assembly of claim 1, wherein the shield is pivotally connected to the collar through a living hinge extending between the shield and the collar.

18. A shieldable needle assembly for administering a unit dose of a vaccine comprising:
a) a unit dose needle assembly comprising:
i) a hub including a needle end and an engagement end; and
ii) a unit dose needle including a prong end configured to hold a unit dose of a vaccine, said unit dose needle extending from the needle end of the hub; and
b) a shield assembly comprising:
i) a collar including a needle engagement end and a handle end;
ii) a shield including a pair of longitudinally extending sidewalls defining a longitudinal opening, said shield pivotably connected to said collar; and
iii) a handle extending from the handle end of said collar,
wherein the needle engagement end of the collar and the engagement end of the hub include corresponding structure for attachment therebetween, and wherein the shield is pivotal with respect to the unit dose needle between a retracted position pivotally spaced from the prong end of the unit dose needle and a shielded position with the prong end of the unit dose needle encompassed within the longitudinal opening.

19. The needle assembly of claim 18, wherein the needle engagement end of the collar and the engagement end of the hub include corresponding threaded surfaces for threaded engagement therebetween.

20. The needle assembly of claim 18, further comprising means for preventing pivotal movement of said shield between the shielded position and the retracted position after the shield has been pivoted to the shielded position.

21. The needle assembly of claim 18, further comprising a packaging needle cover covering said unit dose needle.

22. The needle assembly of claim 18, wherein the shield is pivotally connected to the collar through a hangar bar located on said shield and a hook arm located on said collar whereby said hangar bar engages with said hook arm so that said shield may be pivoted with respect to said collar between said retracted position and said shielded position.

23. The needle assembly of claim 18, wherein the shield is pivotally connected to the collar through a living hinge extending between the shield and the collar.

24. A shieldable, single use unit dose needle assembly for administering a unit dose of a vaccine comprising:
a unit dose needle having a handle end and a prong end configured to hold a unit dose of a vaccine;
a fitting capable of receiving the unit dose needle, said fitting including a hinge having a first end and a second end, the first end being attached to an outer surface of the fitting;
an elongated housing attached to the second end of the hinge, the elongated housing being pivotal and capable of enclosing the unit dose needle, the housing comprising a longitudinal slot for receiving the unit dose needle as the housing is pivoted to enclose the unit dose needle and at least one elongated door member hingedly attached to the housing extending across the longitudinal slot over substantially the entire length of the longitudinal slot, wherein the door member is biased to close the longitudinal slot after a needle has entered the housing through the longitudinal slot; and
a handle extending from a handle end of said fitting.

25. The needle assembly of claim 24, wherein the unit dose needle comprises a bifurcated needle, wherein the prong end includes two pointed prongs which are capable of penetrating or abrading the skin of a patient, and wherein the prongs are separated by a U-shaped channel capable of holding the unit dose of a vaccine.

26. The needle assembly of claim 24, further comprising a hub fixedly attached to the handle end of the unit dose needle for attaching the needle to the fitting.

27. The needle assembly of claim 26, wherein the fitting and the hub include corresponding engaging surfaces for attachment therebetween.

28. The needle assembly of claim 24, wherein the handle includes a profile for accommodating a user's fingers.

29. A shieldable unit dose needle assembly for administering a unit dose of a vaccine comprising:
a collar having opposing first and second ends;
a unit dose needle having a first pronged end and a second end, said pronged end of said unit dose needle extending from said first end of said collar; and
a housing pivotable from said collar to a position in alignment with said unit dose needle for enveloping said unit dose needle.

30. The needle assembly of claim 29, wherein said first pronged end of said unit dose needle comprises at least two pointed tips and a U-shaped channel between said two pointed tips.

31. The needle assembly of claim 29, further comprising means for locking said housing in a position enveloping said unit dose needle.

32. The needle assembly of claim 31, wherein said means for locking is irreversible.

33. The needle assembly of claim 31, wherein said housing comprises means for locking said housing to said collar.

34. The needle assembly of claim 33, wherein said means for locking comprises latches on said housing and detents on said collar for receiving said latches on said collar.

35. The needle assembly of claim 31, wherein said housing comprises means for locking said housing to said unit dose needle.

36. The needle assembly of claim 35, wherein said means for locking comprises a hook extending from an internal surface of said housing towards said unit dose needle.

37. The needle assembly of claim 31, wherein said means for locking comprises first and second locking elements to retain said unit dose needle enveloped within said housing, said first locking element comprising a hook on said housing to hook onto said unit dose needle when aligned with said unit dose needle, and said second locking element comprises a latch on said housing and a detent on said collar for receiving said latch, said second locking element providing locking engagement between said collar and said housing when said latch and said detent are interengaged.

38. The needle assembly of claim 29, further comprising a semi-rigid sleeve detachably matable to said collar for shielding said unit dose needle prior to use.

39. The needle assembly of claim 38, wherein said semi-rigid sleeve provides a hermetically sealed barrier enclosing said unit dose needle between said collar and said semi-rigid sleeve.

40. The needle assembly of claim 38, wherein said assembly is subjected to a sterilization process from the group consisting of e-beam, cobalt, and ethylene oxide sterilization processes.
